# FASCICULE DE BREVET EUROPEEN

(11) **EP 0 517 602 B1**
(45) Date de publication et mention de la délivrance du brevet: **13.04.1994**
(21) Numéro de dépôt: 92401546.4
(22) Date de dépôt: 04.06.1992
(51) Int. Cl.: G01T 1/166, A61B 6/00

(54) **Gamma caméra à deux détecteurs en opposition ayant des mouvements radiaux indépendants**
Gammakamera mit zwei gegenseitigen Detektoren und unabhängigen Radialbewegungen
Gamma camera with two opposite detectors having independent radial movements

(30) Priorité: 07.06.1991 FR 9106961
(43) Date de publication de la demande: 09.12.1992
(73) Titulaire: SOPHA MEDICAL, F-75116 Paris (FR)
(72) Inventeur: Pierfitte, Michel, CABINET BALLOT-SCHMIT, F-75116 Paris (FR)
(74) Mandataire: Schmit, Christian Norbert Marie

(56) Documents cités:
- EP-A- 0 266 846
- EP-A- 0 332 937
- DE-A- 3 145 430
- US-A- 4 649 277
- US-A- 4 652 759
- US-A- 4 692 625

## Description

La présente invention a pour objet une gamma caméra, de préférence avec deux détecteurs opposés ayant des mouvements radiaux indépendants. Dans celle-ci l'ergonomie d'utilisation ainsi que l'efficacité de la détection sont améliorées car les détecteurs sont maintenus de façon indépendante, à la distance la plus faible possible du patient à examiner, améliorant ainsi la résolution.

Des gamma caméras sont par exemple décrites dans le brevet américain de ANGER n° 3 011 057. Une gamma caméra est un appareil comportant une embase, fixe ou tournante ou même mobile par rapport au sol, et qui porte, au bout d'un bras, un détecteur, dit encore tête détectrice. Ce détecteur est muni d'un réseau de tubes photo-multiplicateurs dont les faces d'entrée, juxtaposées les unes aux autres, constituent la surface de détection de la tête détectrice et son champ de détection.

Le principe de l'examen est le suivant. On injecte un produit radioactif dans un patient à examiner. Ce produit est par exemple du Thallium. L'émission radioactive vient exciter un cristal scintillateur du détecteur qui convertit l'énergie des photons gamma en une énergie lumineuse détectable par les tubes photo-multiplicateurs. Le cristal scintillateur est précédé d'un collimateur.

Les scintillations émises sont détectées par les tubes photo-multiplicateurs qui produisent des signaux électriques dépendant de l'intensité lumineuse reçue. En effectuant sur l'ensemble de ces signaux électriques des localisations barycentriques, on peut, d'une manière connue, déterminer la localisation X Y de l'origine la scintillation dans le champ de détection. On réalise alors une acquisition incrémentale en cumulant le nombre de scintillations (ou coups) détectés par élément de localisation dit pixel.

En laissant la tête détectrice dans une position donnée pendant un certain temps au-dessus du corps examiné, on peut alors, pour un angle de vue donné, dit projection, obtenir une image révélatrice de la concentration du produit émetteur dans le corps. Un examen tomographique consiste à acquérir une image par angle de vue, pour un grand nombre d'angles de vue, régulièrement espacés sur un secteur angulaire d'au moins 180°. On sait ensuite, avec des algorithmes de calcul, notamment la rétro-projection filtrée, reconstituer l'image du volume examiné.

Pour augmenter la sensibilité de la caméra, on a pris l'habitude d'utiliser une embase, pouvant éventuellement tourner, munie de deux têtes détectrices au lieu d'une seule. Ces deux têtes sont en vis à vis l'une de l'autre. Quand elles tournent, les têtes tournent ensemble autour du patient examiné.

L'embase est normalement susceptible d'effectuer une rotation sur elle même autour d'un axe de rotation tomographique. La tête détectrice est maintenue par un bras fixé à l'embase par un mécanisme de mouvement radial. Le bras qui supporte la tête décrit au cours de la rotation un cylindre. La tête détectrice est en principe orientée pour que la normale à son champ détecteur, au centre de ce champ, soit perpendiculaire à l'axe de rotation de l'embase. Quand on effectue un mouvement radial, on éloigne, ou on rapproche le bras de cet axe de rotation. Quand on a affaire à une gamma caméra à deux têtes détectrices, celles-ci sont montées chacune sur un bras. Les deux bras sont fixés symétriquement sur l'embase, de part et d'autre de l'axe de rotation. Ils sont déplacés par ailleurs symétriquement autour de cet axe, à la fois en rotation et en mouvement radial. Une telle machine est par exemple décrite dans US-A-4 652 759.

Dans un examen tomographique les deux têtes tournent autour du patient et la valeur du rayon, pour chaque angle de rotation, doit être choisie de façon à ce qu'aucun des détecteurs n'entre en contact avec le patient. Les deux rayons d'écartement des têtes sont identiques. Le patient n'ayant pas une symétrie de révolution, il y a toujours un détecteur qui est plus éloigné du patient que l'autre. Pour celui-ci on perd en résolution. La résolution est moins bonne que si ce détecteur était le plus proche possible du patient.

D'autre part dans un examen dit corps entier, on place le patient sur un lit, et on anime d'un mouvement latéral l'embase portant les deux détecteurs en vis à vis. Compte tenu du mouvement radial symétrique des têtes évoqués ci-dessus, sachant d'une part que la tête inférieure doit garder une altitude, ou une valeur de rayon, constante pour évoluer à une distance fixe du lit, et sachant d'autre part que la tête supérieure doit livrer un passage aux parties les plus proéminentes du patient (en général son ventre), on est obligé de fixer la valeur du rayon durant tout le mouvement latéral, pénalisant ainsi la résolution de la tête supérieure.

L'invention a pour objet de remédier à ces inconvénients en permettant des mouvements radiaux indépendants à chacun des détecteurs. Cette indépendance est apportée par un mouvement radial télescopique de chacun de détecteurs.

L'invention a donc pour objet une Gamma caméra comportant une embase tournante portant deux bras de maintien pour maintenir chacun une tête détectrice placée à l'extrémité d'un bras, ces bras étant mobiles radialement par rapport à l'axe de rotation de l'embase, caractérisée en ce que ces bras comportent des moyens de mouvement télescopique pour provoquer des translations indépendantes de ces têtes perpendiculairement aux bras. gamma caméra

Quand il y a deux têtes les mouvement télescopiques de ces deux têtes peuvent alors être indépendants.

Elle a également pour objet une gamma caméra comportant une embase, deux têtes détectrices, et deux bras de maintien fixés par une de leurs extrémités à l'embase et par l'autre à cette tête, caractérisée en ce que les bras comportent, à leurs extrémités fixées aux têtes, des moyens de mouvement télescopique pour provoquer des translations indépendantes de ces. têtes perpendiculairement aux bras.

L'invention sera mieux comprise à la lecture de la description qui suit et à l'examen des figures qui l'accompagnent, celles-ci ne sont données qu'à titre indicatif et nullement limitatif de l'invention. Les figures montrent :
Figure 1 : une représentation en perspective d'une gamma caméra conforme à l'invention;
Figure 2 : un mode d'utilisation de la gamma caméra de l'invention dans lequel les facilités de mouvement télescopique sont exploitées;
Figure 3 : une représentation schématique d'un mode d'exploration en tomographie dans lequel le mouvement télescopique est exploité;
Figure 4 : un détail montrant le mouvement télescopique.

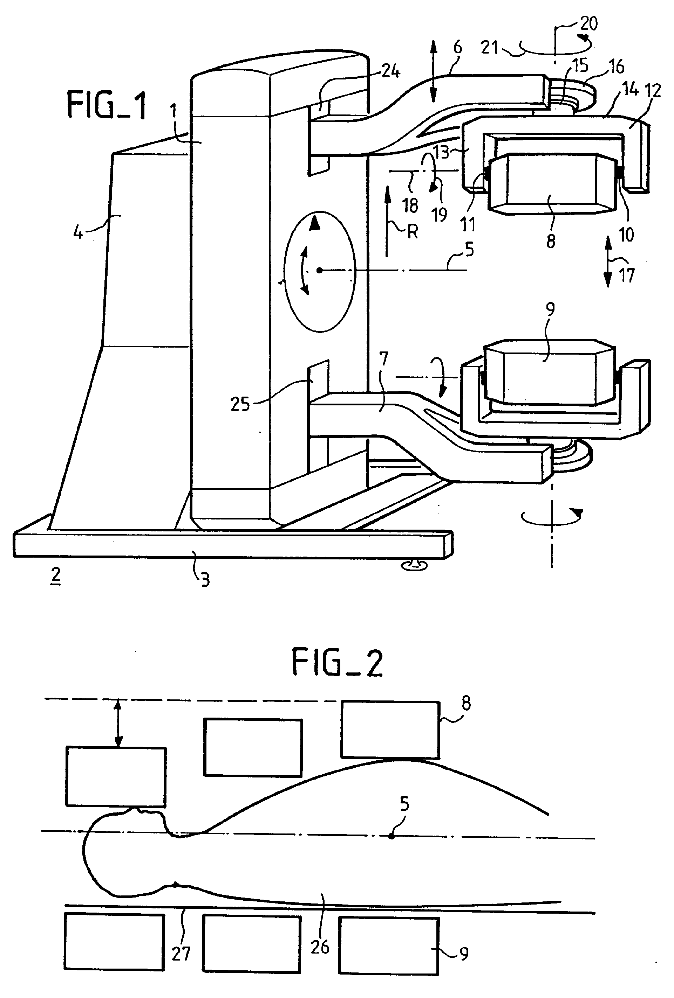

La figure 1 montre une gamma caméra conforme à l'invention. Celle-ci comporte une embase tournante 1 soutenue par un bâti 2 qui comporte un socle 3 maintenant un piédestal 4. Le piédestal 4 comporte essentiellement un arbre, non représenté, permettant à l'embase tournante 1 de tourner autour d'un axe 5 sensiblement horizontal. De l'embase 1 émergent deux bras respectivement 6 et 7 qui maintiennent à leurs extrémités extérieures chacun une tête détectrice respectivement 8 et 9. Les deux têtes sont maintenues à l'extrémité de leur bras de la même façon. Par exemple, la tête 8 est munie de deux axes 10 et 11 qui peuvent tourner dans des paliers en maintenant la tête. Ces paliers eux mêmes sont maintenus dans des flancs respectivement 12 et 13 d'un étrier 14. Le sommet de l'étrier 14 est relié à une couronne 15 qui peut tourner à l'intérieur d'une couronne concentrique 16 qui elle même est fixée à l'extrémité du bras 6.

Un mouvement d'angulation de la tête, effectué autour d'un axe 18 qui passe par les axes et les paliers, est représenté par la flèche 19. Dans une version simplifiée ce mouvement est manuel. Ces mouvement d'angulation sont manuels avec possibilité de sélection parmi des positions prédéfinies. Dans un exemple ces position prédéfinies sont constituées par des encoches réalisées en périphérie de plaques circulaires concentriques à l'axe d'angulation et solidaires de chacune des têtes. Deux crans peuvent venir s'engager dans ces encoches et ainsi maintenir dans des positions prédéfinies l'angulation des têtes.

L'ensemble de l'embase tournante peut tourner autour de l'axe 5 selon un mouvement de rotation 22. La distance R entre l'axe 5 et les bras s'appelle le rayon. Le mouvement radial est celui qui fait varier la valeur de ce rayon. Ce dernier mouvement est déjà connu.

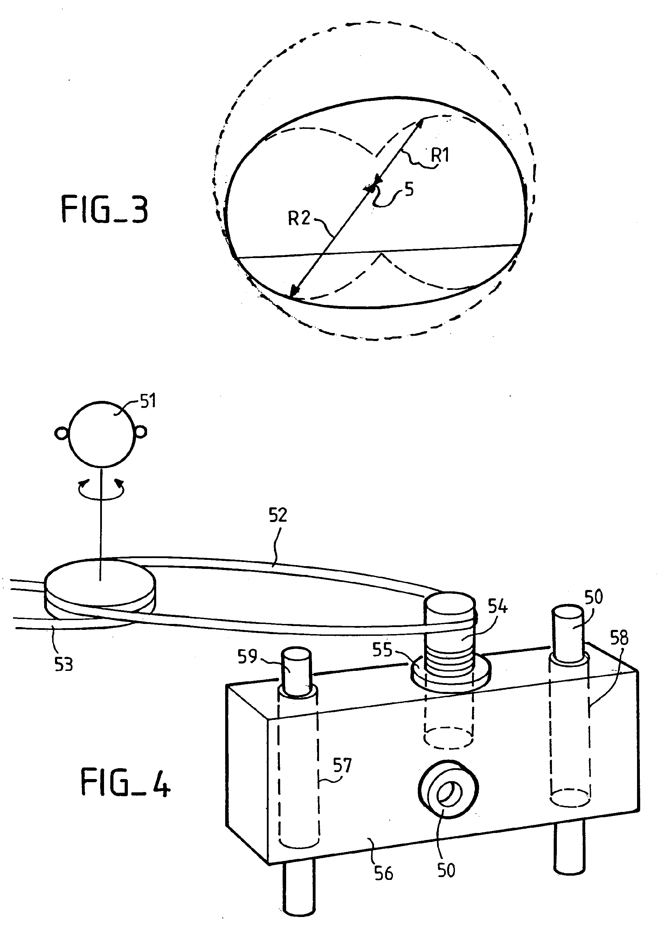

L'étrier 14, qui a une forme de U renversé, comprend, dans chacun des ses flancs 12 et 13, un chariot coulissant, tel que celui montré sur la figure 4, pour permettre aux paliers 50 qui maintiennent les axes 10 et 11 de se mouvoir verticalement. Ce mouvement est de préférence motorisé par un moteur placé dans chaque étrier. Les moteurs sont indépendants. Le moteur 51 est par exemple placé au centre de l'étrier 14. Il entraîne par deux courroies 52 et 53 deux vis sans fin. Seule la vis sans fin 54 est représentée. La vis sans fin 54 se visse dans un écrou 55 solidaire d'un chariot 56. Le chariot 56 est muni de deux douilles 57 et 58 qui coulissent le long de deux arbres 59 et 60. Les arbres 59 et 60 sont maintenus solidaires des flans de l'étrier. La tête de la vis 54 est maintenue fixe en translation mais libre en rotation dans cet étrier. Le mouvement télescopique est représenté symboliquement par la flèche double 17 sur la figure 1.

La figure 2 montre un exemple d'examen dans lequel un patient 26 est couché sur un lit 27. Les deux têtes 8 et 9 sont présentées, d'une manière classique, de part et d'autre du milieu 5 de l'embase tournante. Lorsque le lit 27 est déplacé relativement à droite ou à gauche des têtes, le patient défile entre les deux têtes : on peut effectuer un examen dit corps entier. Au cours de cet examen, on effectue une acquisition permanente. Au fur et à mesure que le tronc du patient passe sous la tête 8 et qu'on arrive à examiner sa tête, on provoque par le mouvement télescopique 17 la descente de la tête supérieure 8 de manière à approcher cette tête détectrice le plus près possible de la tête du patient. En pratique, le mouvement télescopique que l'on sait réaliser est de l'ordre de 8 cm. Dans la position représentée à gauche de la figure 2, la tête supérieure 8 est plus proche de la tête du patient: la résolution en détection est meilleure.

La figure 3 montre d'une manière schématique un examen en tomographie d'un patient non représenté. Dans ce cas alors que le lit est sensiblement parallèle à l'axe 5, on fait tourner la gamma caméra autour du corps du patient. Pour chaque projection, on utilise alors les facultés de mouvement télescopique pour approcher chaque tête le plus près du corps en tenant compte des encombrements présentés par le patient et le lit et de la géométrie d'exploration à centre constant qu'il faut respecter. On obtient avec l'invention une meilleure résolution puisque les têtes peuvent être approchées plus près du corps. Le contour en trait plein représente la trajectoire idéale pour laquelle on utilise au mieux avec l'invention les facultés de mouvement télescopique indépendants, R1 différents de R2. Les trajectoires en tirets montrent la marge de réglage de ces mouvements télescopiques. Le tracé en trait plein est contenu entre les deux tracés en tirets.

## Revendications

1. Gamma caméra comportant une embase tournante (1) portant deux bras (6,7) de maintien pour maintenir chacun une tête (8,9) détectrice placée à l'extrémité d'un bras, ces bras étant mobiles radialement par rapport à l'axe de rotation de l'embase, caractérisée en ce que ces bras comportent des moyens de mouvement télescopique pour provoquer des translations indépendantes de ces têtes perpendiculairement aux bras.

2. Gamma caméra selon la revendication 1, caractérisée en ce que les moyens de mouvement télescopique comportent des moyens pour effectuer une angulation des têtes.

3. Gamma caméra selon la revendication 2, caractérisée en ce que les moyens de mouvement télescopique et d'angulation comportent des étriers munis de deux flancs (12, 13), les têtes des étriers étant fixées aux extrémités des bras, les flancs comportant des paliers rotatifs pour maintenir les têtes en angulation.

4. Gamma caméra selon l'une quelconque des revendications 1 à 2, caractérisée en ce que les moyens de mouvement télescopique comportent un étrier munis de deux flancs, la tête de l'étrier étant fixée à l'extrémité du bras, les flancs comportant des paliers mobiles le long de ces flancs pour permettre le mouvement des têtes.

5. Gamma caméra selon l'une quelconque des revendications 1 à 4, caractérisée en ce que les têtes ont des champs de détection rectangulaires et en ce que les bras possèdent des moyens (20, 21) pour orienter la grande longueur des champs de ces têtes dans une direction quelconque contenue dans un plan perpendiculaire à la direction de mouvement télescopique.

## Patentansprüche

1. Gammakamera mit einem Drehsockel (1), der zwei Haltearme (6, 7) zum Halten je eines Detektorkopfes (8, 9) trägt, der am Ende eines Armes sitzt, wobei die Arme radial bezüglich der Drehachse des Sockels beweglich sind, dadurch gekennzeichnet, daß die Arme Mittel für eine Teleskopbewegung aufweisen, um unabhängige Verschiebungen der Köpfe senkrecht zu den Armen hervorzurufen.

2. Gammakamera nach Anspruch 1, dadurch gekennzeichnet, daß die Mittel für die Teleskopbewegung Mittel zum Durchführen einer Winkelbewegung der Köpfe aufweisen.

3. Gammakamera nach Anspruch 2, dadurch gekennzeichnet, daß die Mittel für die Teleskop- und Winkelbewegungen mit zwei Flanken (12, 13) versehene Bügel aufweisen, wobei die Köpfe der Bügel an den Enden der Arme befestigt sind und die Flanken Drehlager aufweisen, um die Köpfe winkelbeweglich zu halten.

4. Gammakamera nach einem der Ansprüche 1 bis 2, dadurch gekennzeichnet, daß die Mittel für die Teleskopbewegung einen mit zwei Flanken versehenen Bügel aufweisen, wobei der Kopf des Bügels am Ende des Armes befestigt ist und die Flanken bewegliche Lager entlang der Flanken aufweisen, um die Bewegung der Köpfe zu ermöglichen.

5. Gammakamera nach einem der Ansprüche 1 bis 4, dadurch gekennzeichnet, daß die Köpfe rechteckige Detektionsbereiche aufweisen und die Arme Mittel (20, 21) besitzen, um die große Länge der Felder dieser Köpfe in einer beliebigen Richtung zu orientieren, die in einer Ebene senkrecht zu der Richtung der Teleskopbewegung enthalten ist.

## Claims

1. Gamma camera comprising a rotating base (1) bearing two supporting arms (6, 7) each designed to hold a detector head (8, 9) situated at the end of an arm, these arms being radially movable in relation to the rotation axis of the base, characterized in that these arms comprise means for telescopic movement to cause independent traversing movements of the said heads perpendicularly to the arms.

2. Gamma camera according to claim 1, characterized in that means for telescopic movement comprise means for effecting an angulation of the heads.

3. Gamma camera according to claim 2, characterized in that means for telescopic movement and angulation comprise stirrups having two limbs (12, 13), the heads of the stirrups being affixed to the ends of the arms, the limbs having rotating bearings to keep the heads in angulation.

4. Gamma camera according to either of claims 1 to 2 characterised in that the means for telescopic movement comprise a stirrup having two limbs, the head of the stirrup being affixed to the ends of the arms, the limbs comprising bearings movable along the said limbs in order to render possible the movement of the heads.

5. Gamma camera according to any one of claims 1 to 4, characterized in that the heads have rectangular detection fields and that the arms are provided with means (20, 21) to orientate the major length of these fields in any direction contained in a plane perpendicular to the direction of telescopic movement.
